# EUROPÄISCHE PATENTSCHRIFT

(11) **EP 1 345 614 B1**
(45) Veröffentlichungstag und Bekanntmachung des Hinweises auf die Patenterteilung: **11.08.2004**
(21) Anmeldenummer: 01990591.8
(22) Anmeldetag: 21.12.2001
(51) Int. Cl.: A61K 35/78, A61P 17/00

(54) **VERBESSERTER UND STABILER EXTRAKT AUS HYPERICUM PERFORATUM L. , VERFAHREN ZU SEINER HERSTELLUNG UND VERWENDUNG ALS TOPISCHES ARZNEIMITTEL**
IMPROVED AND STABLE EXTRACT FROM HYPERICUM PERFORATUM L. , METHOD FOR THE PRODUCTION THEREOF AND ITS USE AS A TOPICAL MEDICAMENT
EXTRAIT AMELIORE ET PLUS STABLE D'HYPERICUM PERFORATUM L ., PROCEDE PERMETTANT DE LE PRODUIRE ET SON UTILISATION COMME MEDICAMENT TOPIQUE

(30) Priorität: 22.12.2000 DE 10064284; 29.06.2001 DE 10131641
(43) Veröffentlichungstag der Anmeldung: 24.09.2003
(73) Patentinhaber: Dr. Willmar Schwabe GmbH & Co. KG, 76227 Karlsruhe (DE)
(72) Erfinder: KOCH, Egon, 76229 Karlsruhe (DE); ERDELMEIER, Clemens, 76139 Karlsruhe (DE); HERRMANN, Joachim, 68789 St. Leon-Rot (DE)
(74) Vertreter: Adam, Holger, Dr.
(86) Internationale Anmeldenummer: PCT/EP2001/015281
(87) Internationale Veröffentlichungsnummer: WO 2002/051427

(56) Entgegenhaltungen:
- WO-A-00/30660
- DE-A- 19 854 446
- DE-C- 19 619 512
- ORTH H C J ET AL: "STABILITY AND STABILIZATION OF HYPERFORIN" DRUGS MADE IN GERMANY, AULENDORF, DE, Bd. 42, Nr. 4, 1999, Seiten 110-113, XP001056986 ISSN: 0012-6682

## Beschreibung

Die vorliegende Erfindung betrifft einen verbesserten und stabilen (d.h farbstabilen und ggf. hinsichtlich seines Hyperforingehaltes stabilen) Extrakt aus den oberirdischen Teilen von *Hypericum perforatum L.,* ein Verfahren zu seiner Herstellung sowie diesen Extrakt enthaltende pharmazeutische Zubereitungen und topische Arzneimittel, insbesondere Gele, zur Behandlung von Haut- und Schleimhautirritationen und -erkrankungen wie Akne, atopische Dermatitis, Neurodermitis, Psoriasis, Stomatitis, Gürtelrose, Lippenherpes, Warzen, Windpocken, Wunden, Verbrennungen sowie anderen bakteriellen und viralen Haut- und Schleimhautinfektionen und Hauterkrankungen die mit einer Zellproliferation und Entzündung einhergehen.

Hypericumextrakte werden seit vielen Jahren zur Behandlung von nervösen Störungen verwendet. Ihre Verwendung bei Depressionen und psychovegetativen Störungen hat in den vergangenen Jahren als Folge positiver klinischer Studienergebnisse stark zugenommen [W.E.Müller, A. Singer, M.Wonnemann, DAZ 139 (17), 49-58 (1999)]. Die EP-A-0 599 307 beschreibt Primärextrakte aus Johanniskraut. Diese Primärextrakte werden durch einfache Extraktion der Droge mit 96%igem bzw. 60%igem wässrigen Ethanol erhalten. In der DE-A-196 19 512 sowie DE-A-197 14 450 sind Extrakte beschrieben, die einen stabilen Gehalt des ansonsten sehr instabilen Johanniskrautinhaltsstoffs Hyperforin aufweisen. Die WO 99/40905 beschreibt die Verwendung von Johanniskrautextrakten zur Behandlung und Prophylaxe von Demenzerkrankungen. Schließlich werden in der DE-A-199 03 570 Hyperforinzubereitungen beschrieben, in denen das Hyperforin in Form eines Extraktes aus Johanniskraut vorliegt.

Die lokale Applikation von Hypericum-Extrakten - insbesondere in Form von Johanniskraut-Öl - zur Behandlung von Wunden, Ulzera, Verbrennungen, Myalgien, Prellungen etc. hat eine lange Tradition [P. Maisenbacher, Dissertation Uni Tübingen, 1991]. Diese erfahrungsmedizinischen Anwendungen erscheinen auf der Grundlage theoretischer Überlegungen und experimenteller Untersuchungen rational begründet. So gibt es zahlreiche Hinweise, dass Hypericum-Extrakte und einzelne Inhaltsstoffe über antivirale, antibakterielle, antiproliferative und entzündungshemmende Eigenschaften verfügen.

Als maßgebliche Inhaltsstoffe mit antiviraler Aktivität in Hypericum-Extrakten wurden Hypericin und Pseudohypericin identifiziert [EP 0 256 452] [ Lavie, G.; Mazur, Y.; Lavie, D.; Meruelo, D. Med. Res. Rev. 15, (2), 111-119 (1995) [LAVIE, G.; VALENTINE, F.; LEVIN, B.; MAZUR, Y.; GALLO, G.; LAVIE, D.; WEINER, D.; MERUELO, D. Proc. Natl. Acad. Sci. USA 86, (15), 5963-5967 (1989)]. Außer gegenüber Retroviren besitzen diese Napthodianthrone antivirale Eigenschaften auch für andere mit einer Lipidhülle ausgestattete Viren, z.B. Herpesviren. Die antivirale Potenz von Hypericin ist vollständig von einer photodynamischen Aktivierung abhängig bzw. wird unter dem Einfluss von sichtbarem Licht entscheidend verstärkt. Diese Eigenschaft prädestiniert diese Inhaltsstoffe deshalb für die lokale Anwendung bei Virusinfektionen der Haut. Antivirale Wirkungen wurden auch für Aceton- und Wasserextrakte aus Hypericum nachgewiesen, die vorwiegend Catechine und Flavon-Aglyka enthielten [Erdelmeier,C.; Koch,E.; Hörr,R. Hypericum perforatum L. - St. John's Wort, Chemical, Pharmacological and Clinical Aspects, in ,Studies in Natural Products Chemistry', Vol.22, Atta-Ur-Rahman (ed.), Elsevier, Amsterdam, 2000]. Auch von diesen Inhaltsstoffen ist eine therapeutisch relevante antivirale Wirkung in erster Linie bei einer topischen Anwendung zu erwarten. In der Tat zeigte sich jetzt, dass erfindungsgemäß hergestellte Extrakte über antivirale Wirkungen verfügen, die wesentlich stärker ausgeprägt sind, als die von reinem Hypericin (siehe Beispiel 3). Gleichzeitig verfügen solche Extrakte über einen großen therapeutischen Index, der den Unterschied zwischen den erwünschten antiviralen Eigenschaften und unspezifischen zytotoxischen Effekten kennzeichnet.

Antibakterielle Wirkungen von Hypericumextrakten sind sehr gut dokumentiert und werden vor allem auf den Gehalt an Hyperforin zurückgeführt. Hyperforin ist insbesondere wirksam gegenüber Gram-positiven Bakterien und besitzt antibakterielle Eigenschaften z.B. auch für Methicillin-resistente Staphylokokken, die wichtige Erreger von Hautinfektionen darstellen [Schempp, Ch. M.; Pelz, K.; Wittmer, A.; Schoepf, E.; Simon, J. C. Lancet 353, (9170), 2129-2129 (1999)]. Dies erscheint bedeutsam, da z.B. eine enge Korrelation zwischen Streptokokken und Staphylokokken-Infektionen und der Induktion oder dem Wiederaufflammen von Psoriasis besteht. Wie jetzt überraschend gefunden wurde, sind die antibakteriellen Effekte eines erfindungsgemäßen Extraktes aber wesentlich stärker, als aufgrund des Gehaltes an Hyperforin zu erwarten wäre. Das bedeutet, dass die erfindungsgemäßen Extrakte andere wirksame antibakterielle Inhaltsstoffe enthalten oder der Effekt von Hyperforin durch solche Extraktbestandteile verstärkt wird.

Hypericine verfügen neben antiviralen Wirkung auch über antiproliferative Eigenschaften und werden deshalb gegenwärtig klinisch bei Tumorerkrankungen in Kombination mit einer photodynamischen Therapie geprüft. Verantwortlich für die Unterdrückung der Zellproliferation ist höchstwahrscheinlich die Hemmung verschiedener an der intrazellulären Signaltransduktion beteiligter Proteinkinasen. Da auch dieser Effekt lichtabhängig ist, bietet sich für die therapeutische Nutzung dieser Wirkung ebenfalls eine topische Anwendung an. In erster Linie ist hierbei an die Behandlung der Schuppenflechte zu denken, die durch eine Hyperproliferation epidermaler Zellen charakterisiert ist.

Hyperforin besitzt außerdem ausgeprägte antiphlogistische Eigenschaften, indem es z.B. die Einwanderung von neutrophilen Granulozyten in entzündetes Gewebe unterdrückt. Über entzündungshemmende Wirkungen verfügen auch die in den erfindungsgemäßen Hypericumextrakten enthaltenen Flavonoide. Flavonoide verfügen insbesondere über antioxidative Eigenschaften und sind dadurch z.B. in der Lage, die gewebeschädigenden Wirkungen von reaktiven Sauerstoffradikalen, die von Leukozyten gebildet werden, zu unterdrücken. Darüber hinaus ist bekannt, dass Flavonoide eine Vielzahl von Enzymsystemen beeinflussen können, die wichtige zelluläre Reaktionen steuern, die an der Pathogenese der Psoriasis beteiligt sind, wie z.B. Zellproliferation und Immunantwort.

Die Psoriasis ist eine chronische Hauterkrankung, die durch eine Hyperproliferation und abnormale Differenzierung von Keratinozyten sowie eine Entzündung der Epidermis und der Dermis gekennzeichnet ist. Mit den Standardbehandlungsmethoden ist zwar eine vorübergehende Verbesserung der Symptome und auch eine langfristige Kontrolle der Erkrankung zu erreichen, eine komplette Abheilung wird in der Regel aber nicht erzielt. Alle bisher bekannten Therapieansätze weisen außerdem mehr oder weniger schwerwiegende andere Nachteile (unangenehmer Geruch, Hautirritationen, erhöhtes Hautkrebsrisiko, Teratogenität etc.) auf. Es besteht deshalb weiterhin ein dringender Bedarf an wirksamen und nebenwirkungsarmen Methoden zur Behandlung der Psoriasis. Erfindungsgemäß wird dieses Bedürfnis durch die Verwendung von Extrakten aus oberirdischen Teilen von Johanniskraut (*Hypericum perforatum L.*) befriedigt, die wirksame Konzentrationen von Hypericin, Hyperforin und Flavonoiden enthalten.

Eine der meist verbreiteten Hauterkrankungen ist die Akne. Dabei handelt es sich um eine Entzündung der Talgdrüsen, die mit einem Untergang der Talgdrüsenfollikel einhergeht. Als auslösende Ursache der Akne werden androgene Hormone angesehen, die die Sekretproduktion in den Talgdrüsen stimulieren. Wenn gleichzeitig Abflussstörungen auftreten, kann es zu einer bakteriellen Besiedlung des Talgs mit nachfolgender perifollikulärer Entzündung, Abszessbildung, Einschmelzung des Gewebes und einer Fremdkörperreaktion kommen. Es gibt Hinweise, dass in der Haut von Aknekranken das männliche Geschlechtshormon Testosteron unter Einfluss des Enzyms 5α-Reduktase verstärkt in die biologische stärkere Wirkform Dihydrotestosteron umgewandelt wird. Die Hemmung der 5α-Reduktase stellt deshalb eine Möglichkeit zur Behandlung der Akne dar. Es wurde nun überraschend gefunden, dass erfindungsgemäße Extrakte und verschiedene darin enthaltene Inhaltsstoffe eine ausgeprägte Hemmwirkung auf dieses Enzym ausüben und deshalb in Verbindung mit den vorstehend erwähnten antibakteriellen und entzündungshemmenden Eigenschaften in hervorragender Weise zur Behandlung der Akne geeignet sind.

Aus der DE 198 54 446 A1 sowie aus der WO 00/30660 ist die Verwendung von Hyperforin und Hypericin sowie diese Komponenten enthaltende Extrakte als Dermatika bekannt.

Diese Gesamtextrakte werden mit einem Lösungsmittelgemisch bestehend aus Ethanol mit hohem Wasseranteil hergestellt. Erfahrungsgemäß enthalten solche Extrakte hohe Mengen an polyphenolischen Gerbstoffen (Proanthocyanidine), die sich mit der Zeit immer stärker braun verfärben und letztlich die Zubereitung kosmetisch inakzeptabel werden lassen. Dies gilt ebenso für die aus Pflanzenmaterialien in praktisch alle ethanolisch-wässrige Extrakte übergehenden grünen Pigmente (Chlorophylle). Diese Pigmente färben die Extrakte sehr stark und machen sie optisch unansehnlich. Solche Extrakte werden erfahrungsgemäß von Patienten nicht akzeptiert. Dies gilt insbesondere für den Fall der Applikation auf unbedeckte Körperteile.

Die vorstehend beschriebenen Nachteile können mit dem hier beschriebenen erfindungsgemäßen farbstabilen Extrakt eliminiert werden. Überraschenderweise kann durch entsprechende Auswahl des Extraktionsmittels die Extraktion polyphenolischer Gerbstoffe weitgehend unterdrückt werden. Die im resultierenden Primärextrakt vorhandenen grünen Pigmente können durch Filtration über ein Adsorptionsmittel, z.B. ein Adsorberharz nahezu vollständig entfernt werden. Der letztlich erhaltene erfindungsgemäße Extrakt ist kosmetisch-optisch akzeptabel und kann einfach in Salben, Cremes, Gele etc. für externe Zwecke eingearbeitet werden. Der erfindungsgemäße Extrakt ist somit stabil im Sinne von farbstabil, d.h. es kommt nicht zu einer unerwünschten Verfärbung während der Haltbarkeits- und Anwendungszeit des Arzneimittels.

Die Abwesenheit bzw. starke Verringerung des Gehaltes grüner Pigmente (Chlorophylle) und polyphenolischer Gerbstoffe (Proanthocyanidine) kann dadurch nachgewiesen werden, dass die für Chlorophylle typischen Absorptionsbanden im VIS-Spektrum dieses Extraktes zwischen 600 und 700 nm stark vermindert sind oder fehlen, und dadurch, dass HPLC-analytisch keine Signale oder nur sehr stark verminderte Signale sichtbar sind, die auf Proanthocyanidine und Chlorophylle hinweisen.

Die HPLC-Bedingungen für die Bestimmung der Chlorophylle und der Proanthocyanidine sind wie folgt:
Säule: Spherisorb 100 RP-18/Merck (Säulenlänge/Durchmesser: 250/4mm)
Eluens:
A:H₂O 100T./H₃PO₄ 0,3T./Triethylamin 0,2T.
B:Acetonitril (ACN) 100T./H₃PO₄ 0,3T./Triethylamin 0,2T.

Gradient:

| Zeit | Eluens A | Eluens B | Fluss ml/min |
|---|---|---|---|
| 0,0 | 100 | 0 | 1,2 |
| 5,0 | 99 | 1 | 1,2 |
| 55,0 | 60 | 40 | 1,2 |
| 90,0 | 1 | 99 | 1,2 |
| 100,0 | 1 | 99 | 1,2 |
| 105,0 | 100 | 0 | 1,2 |

Fluss: 1, 2 ^{ml}/min
Analysenzeit: 100 min/Gradient
Detektion: UV 2.00-600 nm
Aufgabemenge 20µl ca.5mg/ml
Säulenofen: 25°C

Bei den vorstehend angegebenen HPLC-Bedingungen erscheinen die Chlorophylle bei einer Detektionswellenlänge von 400nm bei einer Retentionszeit im Bereich von 80min bis 100min. Die Proanthocyanidine erscheinen bei einer Detektionswellenlänge von 220nm als breiter Peak bei einer Retentionszeit im Bereich von 20min bis 60min. Die entsprechenden Peaks für die Chlorophylle und Proanthocyanidine treten bei einem 60%-igen wässrigethanolischen Vergleichsextrakt auf und sind stark vermindert oder fehlen bei den erfindungsgemäßen Extrakten.

Bei den erfindungsgemäß verwendeten Extraktionsmitteln handelt es sich um Aceton, Ethanol oder einem Lösungsmittelgemisch aus Aceton und Ethanol. Bevorzugte Extraktionsmittel sind wässriges Aceton, insbesondere ≥ 90%-iges wässriges Aceton, wässriges Ethanol, insbesondere ≥ 90%-iges wässriges Ethanol und ein Gemisch aus beiden Lösungsmitteln, wobei ein Gemisch aus wässrigem Aceton und wässrigem Ethanol in einem Verhältnis von 8:2 bevorzugt ist und ein Gemisch aus 95 Gew.-% Aceton und 92 Gew.-% Ethanol in einem Verhältnis von 8:2 besonders bevorzugt ist.

Als geeignetes Adsorptionsmittel kann in dem erfindungsgemäßen Verfahren z. B. ein Adsorberharz verwendet werden. Beispiele hierfür sind Harze auf Polystyrolbasis, z. B. Diaion HP-Harze der Firma Mitsubishi Kasei Corporation. Ein weiteres Beispiel für ein geeignetes Adsorptionsmittel ist Tonerde, z. B. vom Typ Tonsil der Firma Südchemie AG.

Die erfindungsgemäßen Extrakte aus Hypericum perforatum L. mit abgereichertem Chlorophyllgehalt und mit abgereichertem Gehalt an Proanthocyanidinen weisen vorzugsweise einen Hyperforingehalt von mindestens 2%, einen Gesamthypericingehalt von mindestens 0,2% und einen Gesamtflavongehalt von mindestens 2%, besonders bevorzugt einen Hyperforingehalt von mindestens 4%, einen Gesamthypericingehalt von mindestens 0,4% und einen Gesamtflavongehalt von mindestens 4% und insbesondere einen Hyperforingehalt von 4-8%, einen Gesamthypericingehalt von 0,4-1,0% und einen Gesamtflavongehalt von 4-8% auf.

Bei den erfindungsgemäßen Zubereitungen mit Johanniskrautextrakt handelt es sich insbesondere um topisch zu applizierende, hydrophile oder lipophile homogene, einphasige Zubereitungen in Gelform (s. Beispiele 2a - 2c). Diese Gele sind leicht auf die Haut aufzutragen und zu verreiben. Sie sind insbesondere kosmetisch akzeptabel, d.h. auch bei nicht von Kleidung bedeckten Körperteilen ist nach dem Auftragen keine ungewöhnliche Verfärbung der Haut festzustellen.

Beispiele für geeignete pharmazeutisch übliche Hilfsstoffe zur topischen Applikation und für geeignete übliche Gelgrundlagen sind Polyacrylsäure, Methylcellulose sowie weitere Cellulosederivate.

Des Weiteren weist der erfindungsgemäße Extrakt den Vorteil auf, dass er die pharmakologisch relevanten Inhaltsstoffe Hyperforin, Hypericine und Flavone in idealer Kombination, auch mengenmäßig, enthält. So ist z.B. aufgrund der komplexen Pathogenese der Psoriasis nicht zu erwarten, dass Substanzen mit einem einzelnen, selektiven Wirkmechanismus erfolgreich für die Therapie dieser Erkrankung eingesetzt werden können. Diese Einschätzung wird durch die klinischen Erfahrungen mit den etablierten Therapieverfahren bestätigt. Der erfindungsgemäße Extrakt aus Hypericum zeichnet sich deshalb dadurch aus, dass er verschiedene für die Behandlung der Psoriasis und anderer Hauterkrankungen wichtige Wirkmechanismen (z.B. antiviral, antibakteriell, antiphlogistisch, antiproliferativ) in sich vereinigt, wobei der Kombinationseffekt der einzelnen Komponenten über die Wirkung der Einzelstoffe deutlich hinausgeht (synergistischer Effekt).

Zur Stabilisierung des Hyperforins im Extrakt bzw. der pharmazeutischen Zubereitung können dem Extrakt und/oder der pharmazeutischen Zubereitung (z.B. Gel) Stabilisatoren in einer zur Stabilisierung des Hyperforins ausreichenden Menge zugesetzt werden. Bei den Stabilisatoren handelt es sich um die in der DE-A-196 19 512 beschriebenen Stabilisatoren, die vorzugsweise in einer Konzentration von 0,01% bis 5%, insbesondere 0,2% bis 1%, bezogen auf den Extrakt, vorliegen. Des Weiteren kann es sich bei den Stabilisatoren um die in der DE-A-199 03 570 beschriebenen Komplexierungsmittel in einer zur Komplexierung des Hyperforins ausreichenden Menge handeln. Auf diese beiden Druckschriften wird im Zusammenhang mit der Stabilisierung des Hyperforins ausdrücklich Bezug genommen.

### Beispiel 1: (Herstellung eines erfindungsgemäßen Extraktes)

3,1 kg Hypericum Droge wurden mit der 6,5-fachen Gewichtsmenge Aceton 95 Gew.-% - EtOH 92 Gew.-% 8:2 als Extraktionsmittel 1 min mit dem Ultraturrax homogenisiert (Lichtschutz). Die Lösung wurde danach im Laborextraktionsgefäß 1h bei 50°C extrahiert (N₂-Atmosphäre / Lichtschutz). Zur Extraktlösung wurden 5g Ascorbinsäure zugegeben (1% auf die erwartete Extraktmenge). Die Lösung wurde über eine Filternutsche (Seitz Supra 1500) abgesaugt. Der Drogenrückstand wurde noch zweimal mit der 6-fachen Gewichtsmenge Extraktionsmittel unter den gleichen Bedingungen extrahiert und filtriert.
Zu den vereinigten Filtraten wurden nochmals 5g Ascorbinsäure zugegeben. Die Lösung wird schonend (max.50°C Wärmeband / Lichtschutz) zur Trockene eingeengt.
Ausbeute: 490,02g = 15,8 % (Hyperforingehalt: 5,75% / Hypericingehalt: 0,45%)

Anschließend wurde die Probe in 92Gew%-Ethanol gelöst und durch eine Fritte G2 filtriert. Auf der Fritte blieb ein vernachlässigbarer Rückstand (ca. 2-3g) zurück. 485 g des Extraktes wurden dann auf eine mit Diaion HP-20 gepackte geschlossene Chromatographie-Säule aufgegeben.

### Chromatographiebedingungen:

| | |
|---|---|
| Probe | 485g mit 92Gew.% EtOH auf 15 l verdünnt. |
| Säulengröße | Füllhöhe 42cm; Radius 10cm |
| Säulenfüllung | Diaion HP-20, 0,3-0,8 mm Korngröße |
| Eluens | 1. EtOH 92 Gew.% (100 l; -->Fraktion 1) 2. EtOH 92 Gew.% (40 l; -->Fraktion 2) 2. Aceton 100% (45 l; Chlorophyllfraktion) |
| Fluss | Trennung ca. 1 ^{l}/min |

### Ausbeute:

Die Fraktion 1 enthielt den Hauptteil des Extraktes (412 g = 85%; Hyperforingehalt 6,3 %; Hypericingehalt 0,50 %; Gesamtflavone 6,4%). Diese Fraktion wurde in den Beispielen der Erfindung als erfindungsgemäßer Johanniskrautextrakt verwendet.

Der Extrakt zeigte bei Raumtemperatur und bei 30°C über einen Zeitraum von 6 Monaten keine farbliche Veränderung.

### Beispiel 2a: Hypericumextrakt/Polyacrylat-Gel

| Bestandteil | Mengenanteil (%) |
|---|---|
| Erfindungsgemäßer Johanniskrautextrakt gemäß Beispiel 1 | 2,5 |
| Polyacrylsäure | 1,5 |
| Propylenglykol | 2,5 |
| Tromethaminlösung (40 %, in Wasser) | 5,5 |
| Ethanol 96 Gew.-% | 40,0 |
| Gereinigtes Wasser | 48,0 |

### Herstellung:

Der Gelbildner (Polyacrylsäure, 1 - 3 %, vorzugsweise 1,5 %) wird in einer Mischung aus Wasser, Ethanol und Propylenglykol dispergiert. Der Extrakt (0,5 - 5 %, vorzugsweise 2,5 %) wird hinzugefügt und gemischt. Die Tromethaminlösung wird in kleinen Anteilen hinzugefügt und gemischt. Es bildet sich ein homogenes Gel. Zu dieser Basisrezeptur können Stabilisatoren wie z.B. Ascorbinsäure zugefügt werden.

Die Zubereitung zeigte bei Raumtemperatur und bei 30°C über einen Zeitraum von 6 Monaten keine farbliche Veränderung.

### Beispiel 2b: Hypericumextrakt-Tensidgel

| Bestandteil | Mengenanteil (%) |
|---|---|
| Erfindungsgemäßer Johanniskrautextrakt gemäß Beispiel 1 | 5,0 |
| Macrogol-Glycerol-Hydroxystearat | 35,0 |
| Isopropylmyristat | 10,0 |
| Neutralöl | 10,0 |
| Propylenglykol | 5,0 |
| Gereinigtes Wasser | 35,0 |

### Herstellung:

Der Johanniskrautextrakt, das Tensid (Macrogol-Glycerol-Hydroxystearat), das Isopropylmyristat, das Neutralöl und das Propylenglykol werden homogen gemischt. Anschließend wird das Wasser unter Rühren zugefügt. Es bildet sich ein homogenes Gel. Zu dieser Basisrezeptur können Stabilisatoren wie z.B. Ascorbinsäure zugefügt werden.

Die Zubereitung zeigte bei Raumtemperatur und bei 30°C über einen Zeitraum von 6 Monaten keine farbliche Veränderung.

### Beispiel 2c: Hypericumextrakt-Kohlenwasserstoffgel

| Bestandteil | Mengenanteil (%) |
|---|---|
| Erfindungsgemäßer Johanniskrautextrakt gemäß Beispiel 1 | 5,0 |
| Vaseline | 90,0 |
| Propylenglykol | 5,0 |

### Herstellung:

Die Vaseline wird durch Erwärmen geschmolzen, das Pröpylenglykol zugefügt und gemischt. Der Johanniskrautextrakt wird zugegeben, gemischt und kaltgerührt. Es bildet sich ein homogenes Gel.

Die Zubereitung zeigte bei Raumtemperatur und bei 30°C über einen Zeitraum von 6 Monaten keine farbliche Veränderung.

### Beispiel 3: Antivirale Wirkung

Für die Prüfung auf antivirale Eigenschaften des erfindungsgemäßen Extraktes und von Hypericin wurde der Herpes simplex-Virusstamm 1 (HSV-1), Stamm: McIntyre, ATCC) verwendet.

Zur Beurteilung der "viruziden" Aktivität (Inaktivierungstest) wurden verschiedenen Konzentrationen der Testsubstanzen in einem Volumen von 20 µl mit 980 µl Viruslösung gemischt und nach 1 h Inkubation auf eine Mikrotiterplatte mit 3 Tage alten, konfluenten Verozellen (Affennierenzellen) aufgebracht. Nach Inkubation der Platten für 5 Tage im Brutschrank bei 37°C erfolgte die visuelle Ablesung der Zellkulturen nach HSV-1-spezifischen zytopathogenen Veränderungen (CPE).

Zur Bestimmung der "virostatischen" Aktivität (Inhibitionstest) wurden der erfindungsgemäße Extrakt oder Hypericin in verschiedenen Konzentrationen zu einem geschlossenen Verband von Verozellen in Mikrotiterplatten zugegeben. Nach einer einstündigen Inkubation wurden dann HSV-1 in einer Konzentration von 3000 TCID₅₀/ml zugegeben. Die Platten wurden anschließend bei 37°C und 5% CO₂ im Brutschrank inkubiert. Die Auswertung erfolte nach 48-72 h unter Verwendung von monoklonalen Antikörpern und einer spezifischen Färbemethode.

Zur Beurteilung der zytotoxischen Wirkung wurden Verozellen für 7 Tage mit verschiedenen Konzentrationen von erfindungsgemäßem Extrakt und Hypericin im Brutschrank inkubiert. Die Bestimmung der Zytotoxizität erfolgte dann mit Hilfe des MTT-Tests nach einem publizierten Verfahren (Cinatl J. jr., Cinatl, J., Rabenau, H., Gümbel H.; Doerr, H.W. (1993); "In vitro anti-human immunodeficiency virus activity of 2',3-dideoxynucleotides and their effect on clonal growth of hemopoietic cells from human bone marrow", Arzneimittel-Frsch./Drug Res. 43, 622-625).

Die Auswertung der zytotoxischen Konzentration (TC₅₀) und der virusinhibitorischen Konzentration (IC₅₀) erfolgten mittels linearer Regression. Zur Berechnung des therapeutischen Index wurde die Formel TC₅₀/IC₅₀ eingesetzt.

Ergebnisse der Untersuchungen im "Inaktivierungstest"

| Zubereitung | eingesetzte Konzentration in µg/ml | | | | |
|---|---|---|---|---|---|
| | 100 | 50 | 10 | 1 | 0,5 |
| Erfindungsgemäßer Johanniskrautextrakt gemäß Beispiel 1 | n.b. | n.b. | 3,6x10³ | 8,4x10⁴ | 8,4x10⁶ |
| Hypericin | 4,7x10³ | n.b. | 4.7x10⁶ | n.b. | n.b. |

Die eingesetzte Virusmenge betrug 1,5x10⁷ TCID₅₀. Die "Virusinaktivierung" berechnet sich aus der Differenz der Ausgangsvirusmenge und den oben angegebenen Virustitern. n.b. = nicht bestimmt aufgrund von Zytotoxizität oder der in Vorversuchen bestimmten "virusinaktivierenden" Wirkung.

### Ergebnisse der Untersuchungen im "Inhibitionstest"

| Zubereitung | TC₅₀ (µg/ml) | IC₅₀ (µg/ml) | Therpeutischer Index |
|---|---|---|---|
| Erfindungsgemäßer Johanniskrautextrakt gemäß Beispiel 1 | 1,3 | 0,012 | 108 |
| Hypericin | 74,3 | 2,2 | 34 |

Wie aus den Untersuchungen ersichtlich ist, zeigte Hypericin wie erwartet eine deutliche "virusinaktivierende" Aktivität. Sie betrug bei 100 µg/ml ca. 4 log10-Stufen. Überraschend bewirkte der erfindungsgemäße Extrakt aber eine wesentlich stärkere Wirkung, indem er selbst bei einer Konzentration von 10 µg/ml noch einen ähnlichen starken inhibierenden Effekt ausübt, wie das als wesentlicher antivirale Inhaltsstoff von Johanniskrautextrakten beschriebene Hypericin, das im erfindungsgemäßen Extrakt nur in einem Anteil von etwa 0,5 % enthalten ist. Die potente antivirale Wirksamkeit des erfindungsgemäßen Extraktes wurde im "Inhibitionstest" bestätigt.

### Beispiel 4: Antibakterielle Wirkung

Die antimikrobiellen Wirkungen gegenüber verschiedenen grampositiven Bakterien wurde mit Hilfe der Mikrodilutionsmethode bestimmt. In der Tabelle angegeben sind jeweils die minimale Hemmkonzentration (MHK) und die minimale bakterizide Konzentration (MBK)

| **Bakterien/Pilze** | **Hyperforin** **MHK (µg/ml)** | **Erfindungsgemäßer Extrakt MHK** **(µg/ml )** |
|---|---|---|
| Staphylococcus aureus (SA) | 62,5 | 39,0 |
| Staphylococcus saprophyticus | 62,5-125 | 19,5 |
| Methicillin-resistente SA | 31,25 | 9,8-19,5 |
| Enterococcus faecalis | 125 | 39,0 |
| Enterococcus faecium | 125-250 | 19,5 |
| Enterococcus durans | 125 | 9,8-19,5 |
| Listeria monocytogenes | 15,6-31,25 | 2,5 |
| Streptococcus pyogenes | 15,6 | 2,5-4,9 |

Der erfindungsgemäße Extrakt zeigte an den getesteten grampositiven Bakterien wesentlich stärkere antibakterielle Effekte, als aufgrund des Gehaltes an Hyperforin, das als wesentlicher Inhaltsstoff mit antibakteriellen Wirkungen angesehen wird, zu erwarten wäre.

### Beispiel 5: Antiphlogistische Wirkung

Die antiphlogistischen Eigenschaften des erfindungsgemäßen Hypericumextraktes und von isolierten Inhaltsstoffen wurden im Crotonöl-Ohrödemmodell an männlichen NMRI-Mäusen geprüft. Nach Einleitung einer Narkose durch intraperitoneale Injektion von 60 mg/kg Natrium-Pentobarbital wurden die Testsubstanzen in 10 µl Aceton auf das rechte Ohr aufgetragen, während das linke Ohr zur Kontrolle nur mit 10 µl Acteon behandelt wurde. 30 min später wurde durch Auftragen von 50 µg Crotonöl in 10 µl Aceton auf das linke Ohr eine lokale Entzündung ausgelöst. Nach 6 h wurden die Tiere euthanasiert und die Hemmung der Entzündungsreaktion wurde durch Wiegen eines ausgestanzten Gewebestücks aus beiden Ohren nach folgender Formel quantifiziert: [1-(G_{T}/G_{L})]x100, dabei handelt es sich bei G_{T} bzw. G_{L} um die Gewichtsdifferenz zwischen dem rechten und linken Ohr von Tieren die mit Testsubstanz (G_{T}) bzw. nur dem Lösungsmittel (G_{L}) behandelt wurden.

Die Hemmung der Akkumulation neutrophiler Granulozyten im entzündeten Gewebe wurde durch die Bestimmung von Myeloperoxidase (MPO) in den Ohrhomogenaten ermittelt. Dazu wurden die Biopsieproben in 1 ml Hexadecyltrimethylammoniumbromid (HTAB)-Puffer aufgenommen, mit einem Glashomogenisator bei 4° C homogenisiert und anschließend für 10 sec in einem Sonicator beschallt. Nach einer Zentrifugation bei 4° C mit 3000 g für 10 min wurde der Überstand abgenommen und bis zur Analyse bei - 70° C aufbewahrt. Die MPO-Bestimmung erfolgte in Mikrotiterplatten nach Zugabe von H₂O₂ und dem Indikator O-Dianisidin-Dihydrocholorid als kinetische Messung über einen Zeitraum von 5 min bei 450 nm. Die Auswertung erfolgte gegenüber einer Standardkurve mit Meerrettich-Peroxidase und wurde ermittelt als mU Enzymaktivität pro mg Gewebe.

Die Ergebnisse der Untersuchungen nach Applikation von jeweils 250 µg Testsubstanz bzw. 1000 µg des erfindungsgemäßen Extraktes auf das linke Ohr sind in der nachfolgenden Tabelle zusammengestellt.

| **Inhaltsstoff** | **Hemmung** **Ödem (%)** | **Hemmung** **MPO (%)** |
|---|---|---|
| Erfindungsgemäßer Extrakt | 69 | 72 |
| Amentoflavon | 82 | 70 |
| Biapigenin | 83 | 86 |
| Catechin | 36 | 41 |
| Chlorogensäure | 14 | 16 |
| Epicatechin | 1 | 45 |
| Hyperforin | 65 | 62 |
| Hypericin | 6 | 48 |
| Hyperosid | -27 | 39 |
| Isoquercitrin | -29 | 73 |
| Kämpferol | 91 | 96 |
| Luteolin | 70 | 91 |
| Myricetin | 17 | 75 |
| Quercetin | 82 | 97 |
| Quercitrin | -26 | 23 |
| Rutin | 38 | 55 |

Der erfindungsgemäße Extrakt wurde bei der vorstehend beschriebenen Untersuchung in einer Menge von 1000 µg eingesetzt. Wie sich aus Beispiel 1 ergibt, enthält der verwendete erfindungsgemäße Extrakt nach Beispiel 1 (Fraktion 1) 6,3% Hyperforin als Wirkstoff mit dem mengenmäßig größten Anteil im Extrakt. 1000 µg Extrakt entsprechen somit 63 µg Hyperforin. Die Vergleichstestsubstanzen wiederum wurden in einer Menge von 250 µg verabreicht. Dies bedeutet, dass diese Testsubstanzen in etwa der vierfachen Menge in Bezug auf Hyperforin im Extrakt verabreicht wurden. Trotz dieser Tatsache zeigt der erfindungsgemäße Extrakt im Vergleich zu Hyperforin als Testsubstanz eine deutlich bessere Hemmung. Hierin zeigt sich wiederum der synergistische Effekt zwischen Hyperforin und anderen wirksamkeitsrelevanten Inhaltsstoffen im erfindungsgemäßen Extrakt. Damit wird deutlich, dass für die positiven pharmakologischen Eigenschaften die Summe der Einzelverbindungen wichtig ist, wobei ganz offensichtlich bestimmten Flavonoiden (z.B. Amentoflavon, Biapigenin, Kämpferol, Luteolin, Quercetin) eine besondere Bedeutung zukommt.

### Beispiel 6

### Proliferation von humanen Keratinozyten (HaCaT-Zellen)

Der Einfluss des erfindungsgemäßen Extraktes und verschiedener Inhaltsstoffe auf die Proliferation von humanen Keratinozyten wurde unter Verwendung der Zellinie HaCaT ermittelt. Die Zellen (10.000- 15.000) wurden in 200 µl DMEM mit 5 % fötalem Kälberserum in 96-Well F-Form Mikrotiterplatten ausgesät und für 24 h in Anwesenheit der Testsubstanzen bei 37°C in einem Brutschrank inkubiert. Die Zellproliferation wurde durch Messung des Einbaus von ³H-Methyl-Thymidin (0,5 µCi/Well) während der letzten 6 h in Kultur bestimmt.

| **Inhaltsstoff** | **Konzentration** **(µg/ml)** | **Hemmung** **(%)** |
|---|---|---|
| Erfindungsgemäßer Extrakt gemäß Beispiel 1 | 30 | 81 |
| Amentoflavon | 10 | 68 |
| Biapigenin | 10 | 15 |
| Catechin | 10 | 14 |
| Chlorogensäure | 10 | -9 |
| Epicatechin | 10 | 13 |
| Hyperforin-Na | 10 | 97 |
| Hypericin | 10 | 100 |
| Hyperosid | 10 | -8 |
| Isoquercitrin | 10 | 83 |
| Kämpferol | 10 | 44 |
| Luteolin | 10 | 92 |
| Myricetin | 10 | 26 |
| Quercetin | 10 | -29 |
| Quercitrin | 10 | 35 |
| Rutin | 10 | 9 |

Wie aus den dargestellten Ergebnissen deutlich wird, beruhen die antiproliferativen Eigenschaften des erfindungsgemäßen Extrakts ganz offensichtlich auf der Kombinationswirkung von Hypericin und Hyperforin sowie bestimmter Flavonverbindungen, z.B. Isoquercitrin und Luteolin.

### Beispiel 7: Hemmung der 5α-Reduktase

Untersuchungen zur Hemmung der 5α-Reduktase wurden an Zellen der humanen Prostatakarzinom-Zellinie DU 145 durchgeführt. Die Zellen (250.000) wurden in 5 ml RPMI 1640-Medium mit 10 % fötalem Kälberserum (FKS) in 6-Well Zellkulturplatten ausgesät und bei 37° C in einem Brutschrank inkubiert. Nach 4 Tage wurde das Medium gegen RPMI 1640 mit Zusatz von 10 % Charcoalstripped FKS ausgetauscht. Am folgenden Tag wurden die Testsubstanzen und ¹⁴C-Testosteron zugegeben und weitere 18 h später die Zellüberstände abgenommen. Die Zellüberstände wurden mit 2,5 ml Ethylazetat gemischt und dann 10 min mit 500 g bei Raumtemperatur zentrifugiert. Die organische Phase wurde abgenommen und in ein Glasröhrchen überführt. Die Extraktion wurde anschließend durch erneute Zugabe von 2,5 ml Ethylazetat wiederholt. Die vereinigten organischen Phasen wurden bei 37° C unter N₂-Begasung zur Trockene eingedampft, der Rückstand in 50 µl Ethylazetat aufgenommen und dann auf einer Kieselgel 60 TLC-Platte zweimal mit Ethylazetat/Cyclohexan (1:1) als Laufmittel aufgetrennt. Die gebildeten Testosteron-Metaboliten wurden mit Hilfe eines Linear-Analysers lokalisiert und die Hemmung der 5α-Reduktase anhand des Dihydrotestosteron-Peaks im Vergleich zur Lösungsmittelkontrolle ermittelt.

| **Inhaltsstoff** | **Konzentration** **(µg/ml)** | **Hemmung der** **5α-Reduktase (%)** |
|---|---|---|
| Erfindungsgemäßer Extrakt gemäß Beispiel 1 | 500 | 44 |
| Amentoflavon | 30 | -15 |
| Biapigenin | 30 | -4 |
| Catechin | 30 | -15 |
| Chlorogensäure | 30 | 3 |
| Epicatechin | 30 | -6 |
| Hyperforin-Na | 5 | 10 |
| Hypericin | 30 | -24 |
| Hyperosid | 30 | -1 |
| Isoquercitrin | 30 | -5 |
| Kämpferol | 30 | 12 |
| Luteolin | 30 | 42 |
| Myricetin | 30 | 29 |
| Quercetin | 30 | 46 |
| Quercitrin | 30 | -29 |
| Rutin | 30 | -6 |

Der erfindungsgemäße Extrakt zeigt eine ausgeprägte Hemmwirkung auf die 5α-Reduktase. Die verwendete Konzentration an Testsubstanzen von 30 µg/ml entspricht der Konzentration an Hyperforin, dem mengenmäßig wichtigsten Inhaltsstoff im erfindungsgemäßen Extrakt gemäß Beispiel 1, Fraktion 1.

Im Gegensatz zum erfindungsgemäßen Extrakt (500 µg/ml) erwies sich Hyperforin bei der seinem Anteil am Gesamtextrakt entsprechenden Konzentration von etwa 30 µg/ml als zytotoxisch und wurde deshalb nur bei einer Konzentration von 5 µg/ml geprüft. Aus den Ergebnissen ist zu erkennen, dass selbst bei den geprüften hohen Konzentrationen der Einzelstoffe, die deutlich über ihren jeweiligen Anteil am erfindungsgemäßen Extrakt hinausgehen, keine dieser Verbindungen eine. Wirkung entfaltete, die der des Gesamtextraktes überlegen war. Die Gesamtwirkung des erfindungsgemäßen Extraktes ist deshalb nur mit der Kombinationswirkung der enthaltenen Inhaltsstoffe erklärbar.

### Beispiel 8: (Stabilität der Wirkstoffe, insbesondere Hyperforin in den erfindungsgemäßen Zubereitungen)

Auch das bekanntermaßen chemisch instabile Hyperforin ist in einem erfindungsgemäßen Gel gemäß Beispiel 2a vollständig enthalten, wie durch quantitative Bestimmung des enthaltenen Hyperforins gezeigt werden kann, d.h. bei der Herstellung des Gels gibt es keine Zersetzung.

| Zubereitung | Gemessene Hyperforin-Konzentration (%) | Wiederfindung (%) |
|---|---|---|
| Erfindungsgemäßer Johanniskrautextrakt gemäß Beispiel 1 | 6,33 | -- |
| Erfindungsgemäßer Johanniskrautextrakt gemäß Beispiel 1 / Polyacrylat-Gel | 0,15 | 99,0 |

## Patentansprüche

1. Extrakt aus *Hypericum perforatum L.* mit einem Hyperforingehalt von mindestens 2%, einem Gesamthypericingehalt von mindestens 0,2%, und einem Gesamtflavongehalt von mindestens 2% und mit abgereichertem Chlorophyllgehalt und mit abgereichertem Gehalt an Proanthocyanidinen, wobei die für Chlorophylle typischen Absorptionsbanden im VIS-Spektrum dieses Extraktes zwischen 600 und 700 nm stark vermindert sind oder fehlen, und wobei HPLC-analytisch keine Signale oder nur sehr stark verminderte Signale sichtbar sind, die auf Proanthocyanidine und Chlorophylle hinweisen.

2. Extrakt nach Anspruch 1, **dadurch gekennzeichnet, dass** er einen Hyperforingehalt von mindestens 4%, einen Gesamthypericingehalt von mindestens 0,4%, und einen Gesamtflavongehalt von mindestens 4% aufweist.

3. Extrakt nach Anspruch 1 oder 2, **dadurch gekennzeichnet, dass** er einen Hyperforingehalt von 4-8%, einen Gesamthypericingehalt von 0,4-1,0%, und einen Gesamtflavongehalt von 4-8% aufweist.

4. Extrakt nach einem der Ansprüche 1-3, **dadurch gekennzeichnet, dass** der Extrakt des Weiteren einen Stabilisator in einer Konzentration von 0,01 bis 5 Gew.-% enthält.

5. Extrakt nach Anspruch 4, **dadurch gekennzeichnet, dass** der Stabilisator ausgewählt ist aus der Gruppe bestehend aus Ascorbinsäure, Ascorbinsäureester, organischen Thiolverbindungen, wie Cystein und Glutathion und Komplexierungsmitteln, wie cyclischen Oligosacchariden, Kieselsäuren, Zitronensäure, Zuckeralkoholen, Zellulosederivaten, Polyvinylpyrrolidonen oder Vinylpyrrolidon-Vinylacetat-Copolymeren oder Gemischen davon.

6. Verfahren zur Herstellung eines Extraktes nach einem der Ansprüche 1-5, **dadurch gekennzeichnet, dass** getrocknete Johanniskrautdroge mit Aceton oder Ethanol oder einem Lösungsmittelgemisch aus Aceton und Ethanol extrahiert wird und anschließend Chlorophylle und Proanthocyanidine durch Filtration über ein Adsorberharz abgetrennt werden.

7. Verfahren nach Anspruch 6, **dadurch gekennzeichnet, dass** bei der Extraktion wässriges Aceton oder wässriges Ethanol oder ein Gemisch aus beiden Lösungsmitteln verwendet wird.

8. Verfahren nach Anspruch 7, **dadurch gekennzeichnet, dass** bei der Extraktion ≥90%iges wässriges Aceton oder ≥90%iges wässriges Ethanol oder ein Gemisch aus beiden Lösungsmitteln verwendet wird.

9. Verfahren nach einem der Ansprüche 6-8, **dadurch gekennzeichnet, dass** das acetonische und ethanolische Lösungsmittelgemisch in einem Verhältnis von 8:2 gewählt wird.

10. Verfahren nach einem der Ansprüche 6-9, **dadurch gekennzeichnet, dass** das Adsorberharz ausgewählt ist aus Harzen auf Polystyrolbasis und Tonerde.

11. Verfahren nach einem der Ansprüche 6-10, **dadurch gekennzeichnet, dass** dem Extrakt ein Stabilisator zugesetzt wird.

12. Verfahren nach Anspruch 11, **dadurch gekennzeichnet, dass** der Stabilisator ausgewählt ist aus der Gruppe bestehend aus Ascorbinsäure, Ascorbinsäurederivaten, wie Ascorbinsäureester, organischen Thiolverbindungen, wie Cystein und Glutathion und Komplexierungsmitteln, wie cyclischen Oligosacchariden, Kieselsäuren, Zitronensäure, Zuckeralkoholen, Zellulosederivaten, Polyvinylpyrrolidonen oder Vinylpyrrolidon-Vinylacetat-Copolymeren oder Gemischen davon.

13. Pharmazeutische Zubereitung, enthaltend einen Extrakt nach einem der Ansprüche 1-5 und pharmazeutisch übliche Hilfsstoffe zur topischen Applikation.

14. Gel, enthaltend einen Extrakt nach einem der Ansprüche 1-5 sowie eine Gelgrundlage.

15. Verfahren zur Herstellung einer pharmazeutischen Zubereitung nach Anspruch 13 bzw. eines Gels nach Anspruch 14, **dadurch gekennzeichnet, dass** der Extrakt und die pharmazeutisch üblichen Hilfsstoffe und/oder die Gelgrundlage zusammen mit einem Stabilisator für Hyperforin vermischt werden.

16. Verfahren nach Anspruch 15, **dadurch gekennzeichnet, dass** der Stabilisator ausgewählt ist aus der Gruppe bestehend aus Ascorbinsäure, Ascorbinsäurederivaten, wie Ascorbinsäureester, organischen Thiolverbindungen, wie Cystein und Glutathion und Komplexierungsmitteln, wie cyclischen Oligosacchariden, Kieselsäuren, Zitronensäure, Zuckeralkoholen, Zellulosederivaten, Polyvinylpyrrolidonen oder Vinylpyrrolidon-Vinylacetat-Copolymeren oder Gemischen davon.

17. Extrakt nach einem der Ansprüche 1-5 oder pharmazeutische Zubereitung nach Anspruch 13 oder Gel nach Anspruch 14 zur Anwendung bei der topischen Behandlung von Haut- und Schleimhautirritationen und -erkrankungen, wie Akne, atopische Dermatitis, Neurodermitis, Psoriasis, Stomatitis, Gürtelrose, Lippenherpes, Warzen, Windpocken, Wunden, Verbrennungen sowie anderen bakteriellen und viralen Haut- und Schleimhautinfektionen und Hauterkrankungen die mit einer Zellproliferation und Entzündung einhergehen.

## Claims

1. Extract of *Hypericum perforatum L.* with a hyperforin content of at least 2%, a total hypericin content of at least 0.2% and a total flavone content of at least 2% and with reduced chlorophyll and proanthocyanidines content, wherein the absorption bands in the VIS spectrum typical of chlorophylls are greatly reduced or absent altogether between 600 and 700 nm, and wherein no signals or only greatly-reduced signals indicating proanthocyanidines and chlorophylls are visible in an HPLC analysis.

2. Extract according to claim 1, **characterised in that** it shows a hyperforin content of at least 4%, a total hypericin content of at least 0.4% and a total flavone content of at least 4%.

3. Extract according to claim 1 or 2, **characterised in that** it shows a hyperforin content of 4-8%, a total hypericin content of at least 0.4%-1.0% and a total flavone content of 4-8%.

4. Extract according to any one of claims 1 to 3, **characterised in that** the extract in addition contains a stabiliser in an amount of 0.01 to 5% by weight.

5. Extract according to claim 4, **characterised in that** the stabiliser is selected from the group consisting of ascorbic acid, ascorbic-acid esters, organic thiol compounds such as cysteine and glutathion, and complexing agents such as cyclic oligo-saccharides, silicic acids, citric acid, sugar alcohols, cellulose derivatives, polyvinyl-pyrrolidones or vinyl-pyrrolidone-vinylacetate-copolymers, or mixtures thereof.

6. Method for the preparation of an extract according to any one of claims 1 to 5, **characterised in that** dried St. John's wort drug is extracted with acetone or ethanol, or a solvent mixture containing acetone and ethanol, and chlorophylls and proanthocyanidines are then separated out by filtration using a suitable adsorbing resin.

7. Method according to claim 6, **characterised in that** aqueous acetone or aqueous ethanol, or a mixture of both solvents, is deployed for extraction.

8. Method according to claim 7, **characterised in that** ≥90% aqueous acetone or ≥90% aqueous ethanol, or a mixture of both solvents, is deployed for extraction.

9. Method according to any one of claims 6 to 8, **characterised in that** the acetone and ethanol solvent mixture is selected in the ratio of 8:2.

10. Method according to any one of claims 6 to 9, **characterised in that** the adsorbing resin is selected from resins based on polystyrol and argillaceous earth.

11. Method according to any one of claims 6 to 10, **characterised in that** a stabiliser is added to the extract.

12. Method according to claim 11, **characterised in that** the stabiliser is selected from the group consisting of ascorbic acid, ascorbic-acid derivatives such as ascorbic-acid esters, organic thiol compounds such as cysteine and glutathion, and complexing agents such as cyclic oligo-saccharides, silicic acids, citric acid, sugar alcohols, cellulose derivatives, polyvinyl-pyrrolidones or vinyl-pyrrolidone-vinylacetate-copolymers, or mixtures thereof.

13. Pharmaceutical preparation containing an extract according to any one of claims 1 to 5, together with conventional pharmaceutically acceptable excipients for topical application.

14. Gel containing an extract according to any one of claims 1 to 5 as well as a gel foundation.

15. Method for the preparation of a pharmaceutical preparation according to claim 13, or a gel according to claim 14, **characterised in that** the extract and the conventional pharmaceutically acceptable excipients and/or the gel foundation are blended together with a hyperforin stabiliser.

16. Method according to claim 15, **characterised in that** the stabiliser is selected from the group consisting of ascorbic acid, ascorbic-acid derivatives such as ascorbic-acid esters, organic thiol compounds such as cysteine and glutathion, and complexing agents such as cyclic oligo-saccharides, silicic acids, citric acid, sugar alcohols, cellulose derivatives, polyvinyl-pyrrolidones or vinyl-pyrrolidone-vinylacetate-copolymers, or mixtures thereof.

17. Extract according to any one of claims 1 to 5, or a pharmaceutical preparation according to claim 13, or a gel according to claim 14, for the use for topical treatment of skin and mucous membrane irritations and disorders such as acne, atopic dermatitis, neuro-dermatitis, psoriasis, stomatitis, herpes zoster, herpes labialis (lip herpes), warts, varicella (chickenpox), sores, burns, as well as other bacterial or viral skin and mucous membrane infections that are accompanied by cell proliferation and inflammation.

## Revendications

1. Extrait de *Hypericum perforatum L.* avec une teneur en hyperforine d'au moins 2 %, une teneur globale en hypericine d'au moins 0,2 %, et une teneur totale en flavonoïde d'au moins 2 %, et avec une teneur appauvrie en chlorophylle et avec une teneur appauvrie en proanthocyanidines, où les bandes d'absorption typiques de la chlorophylle dans le spectre visible de cet extrait entre 600 et 700 nm sont fortement diminuées ou manquantes et où par analyse HPLC, aucun signal ou des signaux seulement très fortement diminués sont visibles, qui indiquent les proanthocyanidines et la chlorophylle.

2. Extrait selon la revendication 1, **caractérisé en ce qu'**il présente une teneur en hyperforine d'au moins 4 %, une teneur globale en hypericine d'au moins 0,4 %, et une teneur totale en flavonoïde d'au moins 4 %.

3. Extrait selon la revendication 1 ou 2, **caractérisé en ce qu'**il présente une teneur en hyperforine de 4-8 %, une teneur globale en hypericine de 0,4-1,0 %, et une teneur totale en flavonoïde de 4-8 %.

4. Extrait selon l'une quelconque des revendications 1 à 3, **caractérisé en ce que** l'extrait contient par ailleurs, un stabilisant en une concentration de 0,01 à 5 % en poids.

5. Extrait selon la revendication 4, **caractérisé en ce que** le stabilisant est choisi parmi le groupe consistant en l'acide ascorbique, un ester de l'acide ascorbique, des composés thiol organiques, comme la cystéine et la glutathione et des agents de complexation, comme des oligosaccharides cycliques, des acides siliciques, l'acide citrique, des alcools de sucre, des dérivés de la cellulose, des polyvinylpyrrolidones ou des copolymères vinylpyrrolidone-acétate de vinyle ou leurs mélanges.

6. Procédé de préparation d'un extrait selon l'une quelconque des revendications 1 à 5, **caractérisé en ce que** le millepertuis séché est extrait avec de l'acétone, de l'éthanol ou un mélange d'acétone et d'éthanol et ensuite, la chlorophylle et les proanthocyanidines sont séparées par filtration sur une résine d'adsorption.

7. Procédé selon la revendication 6, **caractérisé en ce que** lors de l'extraction, on utilise de l'acétone aqueux ou de l'éthanol aqueux ou un mélange des deux solvants.

8. Procédé selon la revendication 7, **caractérisé en ce que** lors de l'extraction, on utilise de l'acétone aqueux à ≥ 90 % ou de l'éthanol aqueux à ≥ 90 % ou un mélange des deux solvants.

9. Procédé selon l'une quelconque des revendications 6 à 8, **caractérisé en ce que** le mélange de solvants acétonique et éthanolique est choisi en un rapport de 8:2.

10. Procédé selon l'une quelconque des revendications 6 à 9, **caractérisé en ce que** la résine adsorbante est choisie parmi les résines à base de polystyrène et d'alumine.

11. Procédé selon l'une quelconque des revendications 6 à 10, **caractérisé en ce que** l'on ajoute un stabilisant à l'extrait.

12. Procédé selon la revendication 11, **caractérisé en ce que** le stabilisant est choisi parmi le groupe consistant en l'acide ascorbique, des dérivés de l'acide ascorbique, comme un ester de l'acide ascorbique, des composés thiol organiques, comme la cystéine et la glutathione et des agents de complexation, comme des oligosaccharides cycliques, des acides siliciques, l'acide citrique, des alcools de sucre, des dérivés de la cellulose, des polyvinylpyrrolidones ou des copolymères vinylpyrrolidone-acétate de vinyle ou leurs mélanges.

13. Composition pharmaceutique, contenant un extrait selon l'une quelconque des revendications 1 à 5, et des auxiliaires pharmaceutiques usuels pour application topique.

14. Gel, contenant un extrait selon l'une quelconque des revendications 1 à 5, ainsi qu'une base de gel.

15. Procédé de préparation d'une composition pharmaceutique selon la revendication 13 ou d'un gel selon la revendication 14, **caractérisé en ce que** l'on mélange l'extrait et les auxiliaires pharmaceutiques usuels et/ou la base de gel, avec un stabilisant pour l'hyperforine.

16. Procédé selon la revendication 15, **caractérisé en ce que** le stabilisant est choisi parmi le groupe consistant en l'acide ascorbique, des dérivés de l'acide ascorbique, comme un ester de l'acide ascorbique, des composés thiol organiques, comme la cystéine et la glutathione et des agents de complexation, comme des oligosaccharides cycliques, des acides siliciques, l'acide citrique, des alcools de sucre, des dérivés de la cellulose, des polyvinylpyrrolidones ou des copolymères vinylpyrrolidone-acétate de vinyle ou leurs mélanges.

17. Extrait selon l'une quelconque des revendications 1 à 5 ou composition pharmaceutique selon la revendication 13 ou d'un gel selon la revendication 14, pour une utilisation pour le traitement topique d'irritations et de maladies de la peau et des muqueuses, comme l'acné, la dermatite atopique, la neurodermite, le psoriasis, la stomatite, le zone, le bouton de fièvre, les verrues, la varicelle, les lésions, les brûlures ainsi que d'autres infections bactériennes et virales de la peau et des muqueuses et de maladies de la peau, qui se développent avec une prolifération cellulaire et une inflammation.
